# EUROPEAN PATENT APPLICATION

(11) **EP 4 807 760 A1**
(43) Date of publication of application: **16.09.2026**
(21) Application number: 25190290.4
(22) Date of filing: 17.07.2025
(51) Int. Cl.: G16H 50/50, A61C 19/06

(54) **METHOD, APPARATUS, AND COMPUTER-READABLE RECORDING MEDIUM FOR DESIGNING MOUTH SHIELD HAVING MEDICAMENT SUPPLY UNIT**

(30) Priority: 10.03.2025 KR 20250030550
(71) Applicant: Innodtech, Inc., Gwangju (KR)
(72) Inventor: JOO, Bo Hoon, Seoul (KR)
(74) Representative: Kaya, Erdem

(57) **Abstract**

The present invention relates to a method for designing a mouth shield having a medicament supply unit, comprising: a temporary model generation step (S10) of identifying a tooth part (30T) and a gum part (30G) using a preset object extraction algorithm from oral scan data (1), which is obtained by three-dimensionally scanning an oral condition of a subject, and generating a temporary model by performing modeling on the mouth shield having a shape corresponding to the identified tooth part (30T) and gum part (30G) for the subject using a design program; and a definitive model generation step (S20) of generating a definitive model by performing correction on the temporary model generated in the temporary model generation step (S10), in which the definitive model is generated by forming continuous concave groove parts (H) in an inner region including at least one of a lingual side and a labial side of the temporary model along an arrangement direction of teeth.

## Description

### BACKGROUND OF THE INVENTION

### 1. Field of the Invention

The present invention relates to a method for designing a mouth shield having a medicament supply unit, and more specifically, to a technology of designing a mouth shield to achieve the purpose of enhancing health by a medicament and improve wearing sensation and durability in an oral cavity by continuously supplying the medicament, which is prepared to enhance oral health in the oral cavity, to a specific site.

### 2. Description of the Related Art

Currently, various types of technologies for delivering a medicament into an oral cavity are being developed and commercialized in the field of dentistry and oral treatment.

Examples of the technologies largely include an oral rinsing solution-based medicament delivery method and an application-type medicament-based medicament delivery method, in which the oral rinsing solution-based medicament delivery method is a method in which a user keeps an oral rinsing solution containing ingredients such as an antibacterial agent, fluorine, and an anti-inflammatory agent in the oral cavity for a certain time and then spits the oral rinsing solution out to remove bacteria in the oral cavity and prevent periodontal diseases. However, the oral rinsing solution-based medicament delivery method has problems in which repeated use of the oral rinsing solution is required since the medicament ingredients do not stay in the oral cavity for a long time and are rapidly removed together with saliva, the effect disappears after a predetermined time, and it is difficult to intensively deliver the medicament to a measurement site in the oral cavity.

In addition, the application-type medicament-based medicament delivery method is a method of directly applying fluorogel, antibiotic ointment, analgesic gel, or the like to a specific site with a finger or a cotton swab. However, the method has a problem in which the fluorogel, antibiotic ointment, analgesic gel, or the like is easily diluted or removed by saliva and food in the oral cavity, resulting in a limitation that the treatment effect is not significant because continuous application thereof is also required.

In order to overcome the limitations, Korean Registered Patent No. 10-2569589 (oral sealing wrap having elasticity) discloses a technology for delivering a patch-type medicament that is a product for oral care and includes a layer having a surface in contact with teeth or tissues around the teeth, and allows the medicament applied to a target site in the oral cavity to stay for a desired time.

However, in the case of the prior art, there may be a problem that the oral sealing wrap easily falls before a target time according to an oral environment (amount of saliva secretion, habit) of the user, and there is a limitation in that the medicament delivery effect is only insignificant because the medicament is not sufficiently applied to the target site due to the anatomical structure of the teeth or gums different for each user.

### SUMMARY OF THE INVENTION

Accordingly, an object of the present invention is to achieve the purpose of enhancing oral health by continuously supplying a medicament, which is prepared to enhance oral health in an oral cavity, to a specific site.

In addition, another object of the present invention is to provide a mouth shield that is directly worn in the oral cavity, so that appropriate elasticity and strength are ensured by applying a multi-layer structure instead of a simple single-layer structure, thereby improving wearing sensation and durability.

To achieve the above-described objects, according to one embodiment of the present invention, the present invention relates to a method for designing a mouth shield having a medicament supply unit, which is implemented by a computing device including one or more processors and one or more memories for storing instructions executable in the processors. More specifically, the method includes: a temporary model generation step of identifying a tooth part and a gum part using a preset object extraction algorithm from oral scan data, which is obtained by three-dimensionally scanning an oral condition of a subject, and generating a temporary model by performing modeling on the mouth shield having a shape corresponding to the identified tooth part and gum part for the subject using a design program; and a definitive model generation step of generating a definitive model by performing correction on the temporary model generated in the temporary model generation step, in which the definitive model is generated by forming continuous concave groove parts in an inner region including at least one of a lingual side and a labial side of the temporary model along an arrangement direction of teeth, in which design data for the definitive model generated in the definitive model generation step is provided to a 3D printing laboratory, in which, in the definitive model generation step, when the concave groove parts are formed, the concave groove parts are formed based on a gingival margin line corresponding to a boundary site between the tooth part and the gum part in the temporary model, and in which when the concave groove parts are formed, a required amount of a medicament is calculated in consideration of factors including at least one of age, body weight, gum condition, and severity of a disease of the subject, and a concave groove part having a size corresponding to the calculated required amount of the medicament is designed.

In this case, the method for designing a mouth shield having a medicament supply unit may further include a dedicated tube designing step of designing a dedicated tube that is designed in a shape corresponding to the concave groove part as the medicament supply unit and has a discharge structure for discharging a gel-type medicament that is filled in the dedicated tube, after the definitive model generation step, so that the design data for the definitive model and design data for the dedicated tube may be provided to the 3D printing laboratory to manufacture a real mouth shield and a dedicated tube that enables filling of the gel-type medicament.

In addition, in the dedicated tube designing step, the medicament may be automatically designed such that, after the dedicated tube is filled with the gel-type medicament, the dedicated tube filled with the medicament may match a shape of the concave groove part to allow the dedicated tube to be detachably installed on the concave groove part, and the dedicated tube may be designed to include at least one of a first dedicated tube formed on an outer surface thereof with a plurality of perforated holes serving as a discharge path for discharging a medicament filled in the dedicated tube such that the filled medicament is discharged, a second dedicated tube designed based on the first dedicated tube, in which the perforated holes are additionally provided with a thin film-type elastic membrane so that when the subject wears the mouth shield provided with the dedicated tube in the oral cavity, the thin film-type elastic membrane is deformed due to pressure inside the oral cavity so that the filled medicament is discharged, and a third dedicated tube designed based on the first dedicated tube, in which the outer surface of the dedicated tube is sealed with a dissolving film so that when the subject wears the mouth shield provided with the dedicated tube in the oral cavity, the dissolving film is dissolved so that the filled medicament is discharged through the perforated holes.

In addition, in the temporary model generation step, as a material of designing the temporary model, a urethane-based material may be used in a form of multi-layers, in which the multi-layers may include a first layer configured to directly surround an outer surface of the tooth part of the subject, and a second layer configured to surround an outer surface of the first layer, the first layer may be formed of a urethane-based material having elastic modulus and hardness that are relatively lower than elastic modulus and hardness of the second layer, and the second layer may be formed of a urethane-based material having elastic modulus and hardness that are relatively higher than the elastic modulus and hardness of the first layer.

In addition, in the definitive model generation step, mutually different design factors may be applied for each region of an inside surface of the concave groove part, a low friction coating may be applied to an inlet region of the concave groove part, and a high friction protrusion pattern may be applied to an inner region of the concave groove part.

Meanwhile, an apparatus for designing a mouth shield having a medicament supply unit, which is implemented by a computing device including one or more processors and one or more memories for storing instructions executable in the processors, includes: a temporary model generation unit configured to identify a tooth part and a gum part using a preset object extraction algorithm from oral scan data, which is obtained by three-dimensionally scanning an oral condition of a subject, and generate a temporary model by performing modeling on the mouth shield having a shape corresponding to the identified tooth part and gum part for the subject using a design program; and a definitive model generation unit configured to generate a definitive model by performing correction on the temporary model generated by the temporary model generation unit, in which the definitive model is generated by forming continuous concave groove parts in an inner region of the temporary model along an arrangement direction of teeth, in which design data for the definitive model generated by the definitive model generation unit is provided to a 3D printing laboratory, the definitive model generation unit forms the concave groove parts based on a gingival margin line corresponding to a boundary site between the tooth part and the gum part in the temporary model, when the concave groove parts are formed, and when the concave groove parts are formed, a required amount of a medicament is calculated in consideration of factors including at least one of age, body weight, gum condition, and severity of a disease of the subject, and a concave groove having a size corresponding to the calculated required amount of the medicament is designed.

Meanwhile, a computer-readable recording medium that stores instructions for allowing a computing device to perform the following steps, in which the steps include: a temporary model generation step of identifying a tooth part and a gum part using a preset object extraction algorithm from oral scan data, which is obtained by three-dimensionally scanning an oral condition of a subject, and generating a temporary model by performing modeling on the mouth shield having a shape corresponding to the identified tooth part and gum part for the subject using a design program; and a definitive model generation step of generating a definitive model by performing correction on the temporary model generated in the temporary model generation step, in which the definitive model is generated by forming continuous concave groove parts in an inner region including at least one of a lingual side and a labial side of the temporary model along an arrangement direction of teeth, in which design data for the definitive model generated in the definitive model generation step is provided to a 3D printing laboratory, in which, in the definitive model generation step, when the concave groove parts are formed, the concave groove parts are formed based on a gingival margin line corresponding to a boundary site between the tooth part and the gum part in the temporary model, and when the concave groove parts are formed, a required amount of a medicament is calculated in consideration of factors including at least one of age, body weight, gum condition, and severity of a disease of the subject, and a concave groove having a size corresponding to the calculated required amount of the medicament is designed.

According to one embodiment of the present invention, a medicament that is prepared to enhance oral health in the oral cavity is continuously supplied to a specific site, so that a treatment effect may be maintained for a long time compared to a simple gargle or ointment application method, and accordingly, the purpose of enhancing oral health may be achieved rapidly.

In addition, according to one embodiment of the present invention, an efficient local treatment effect may be expected by forming the concave groove part formed in the mouth shield along the gingival margin line, and an excessive supply of a medicament and an appropriate treatment effect may be expected by adjusting the amount and discharge rate of the medicament.

In addition, according to one embodiment of the present invention, a material of the mouth shield is implemented in a form of multi-layers formed of soft urethane and hard urethane, it is possible to expect improvement in wearing sensation and durability of the mouth shield.

In addition, according to one embodiment of the present invention, it is possible to provide an optimal treatment solution by enabling customized manufacture of a mouth shield reflecting an oral condition and an oral structure of a subject, and to provide a mouth shield having various applicability, from a preventive function of tooth decay prevention, treatment of periodontal disease, pain relief, and whitening treatment, to a cosmetic purpose, treatment of a disease, and the like, according to the characteristics of the medicament.

### BRIEF DESCRIPTION OF THE DRAWINGS

FIGS. 1 and 2 are flowcharts of a method for designing a mouth shield having a medicament supply unit according to one embodiment of the present invention.
FIG. 3 is a view showing an example of identifying objects of a tooth part and a gum part in oral scan data according to one embodiment of the present invention.
FIG. 4 is a view showing an example for a structure of a mouth shield provided in a form of multi-layers according to one embodiment of the present invention.
FIG. 5 is a view showing an example of wearing the mouth shield having the medicament supply unit according to one embodiment of the present invention.
FIG. 6 is a view showing an example for structures of a concave groove part and a dedicated tube according to one embodiment of the present invention.
FIG. 7 is a configuration view of an apparatus for designing a mouth shield having a medicament supply unit according to one embodiment of the present invention.
FIG. 8 is a view showing an example of an internal configuration of a computing device according to one embodiment of the present invention.

### DETAILED DESCRIPTION OF THE INVENTION

Hereinafter, various embodiments and/or aspects will be disclosed with reference to drawings. In the following description, multiple concrete details will be disclosed in order to help general understanding of one or more aspects for the purpose of description. However, it will also be appreciated by those skilled in the art to which the present invention pertains that such aspect(s) may be practiced without the specific details. In the following description and accompanying drawings, specific exemplary aspects of one or more aspects will be described in detail. However, the aspects are exemplary, and some equivalents of various aspects may be used, and the descriptions herein are intended to include both the aspects and equivalents thereto.

It is not intended that any "embodiment", "example", "aspect", "illustration", and the like used in the specification is preferable or advantageous over any other "embodiment", "example", "aspect", "illustration", and the like.

Further, the terms "includes" and/or "including" mean that a corresponding feature/or component exists, but it should be appreciated that the terms "include" or "including" mean that presence or addition of one or more other features, components, and/or a group thereof is not excluded.

Further, terms including an ordinal number such as "first" or "second' may be used for the names of various components, not limiting the components. The above terms are used merely for the purpose of distinguishing one element from another element. For example, a first component may be referred to as a second component and vice versa without departing the scope of the present invention. The term "and/or" includes a combination of a plurality of related enumerated items or any of the plurality of related enumerated items.

In addition, unless otherwise defined, all terms used herein, including technical or scientific terms, have the same meaning as commonly understood by one of ordinary skill in the art to which this invention belongs. Terms as those defined in generally used dictionaries are to be interpreted to have the meanings consistent with the contextual meanings in the relevant field of art, and are not to be interpreted to have idealistic or excessively formalistic meanings unless explicitly defined in the embodiments of the present invention.

The present invention relates to a method for designing a mouth shield having a medicament supply unit. Specifically, one object of the present invention is to achieve the purpose of enhancing oral health by continuously supplying the prepared medicament to a specific site in order to enhance oral health in an oral cavity, and another object of the present invention is to provide a mouth shield directly worn in the oral cavity, in which the mouth shield is formed to secure appropriate elasticity and strength by applying a multi-layer structure instead of a simple single-layer structure, thereby improving wearing sensation and durability.

Meanwhile, it should be understood that the present invention is a technology related to a structure and design system of the mouth shield, and does not include clinical treatment and does not include medical treatment or clinical treatment and methods for humans.

Hereinafter, a detailed description of the present invention for achieving the above object will be described with reference to the accompanying drawings, and a plurality of drawings may be referenced simultaneously to describe one or more technical features or components constituting the present invention.

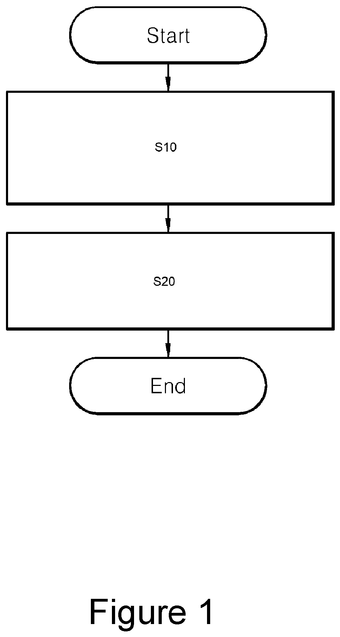

First, referring to FIG. 1, it can be seen that FIG. 1 is a flowchart showing a method for designing a mouth shield (30)having a medicament supply unit. As shown in FIG. 1, according to the present invention, a temporary model generation step (S10)of identifying a tooth part (30T) and a gum part (30G) using a preset object extraction algorithm from oral scan data (1), which is obtained by three-dimensionally scanning an oral condition of a subject, and generating a temporary model by performing modeling on the mouth shield (30)having a shape corresponding to the identified tooth part (30T) and gum part (30G) for the subject using a design program, is performed.

Specifically, the oral scan data (1) described in a temporary model generation step (S10) may be understood as a concept of data obtained by scanning the oral cavity of the subject using a 3D oral cavity scanner (intraoral scanner, CBCT, etc.), and a file format thereof may be an STL file format and may follow a file format having the best compatibility in a 3D graphic software.

In addition, the oral scan data (1) includes information about teeth, gums, and oral structures, and according to the present invention, the object extraction algorithm is applied to the oral scan data (1) to identify the tooth part (30T) and the gum part (30G) of the subject.

As one embodiment, the object extraction algorithm used in the present invention undergoes a process of locating a circle at the center of a simple coordinate of an oral scan data image and gradually increasing the size of the circle to search for a contact point for initial contact with the teeth or gums. Accordingly, the object extraction algorithm may scatter microspheres at regular intervals based on the initial contact point so that the microspheres may be located in each tooth, thereby identifying the location and outline of the teeth and securing basic data capable of searching for boundaries between the teeth and the gums.

Meanwhile, after searching for the initial contact point, a process of arranging one or more spheres (sizes of 1/10,000 mm) at regular intervals inside the teeth, detecting an event in which the spheres are randomly moved at a constant speed to escape from the inside of the teeth to the outside, and replicating the spheres at regular cycles to uniformly distribute the spheres inside the teeth, is performed.

However, in this case, an STL file-based three-dimensional model is formed of triangular meshes so that a mesh data direction is reversed, a relationship between the inside and the outside of the teeth is clearly set by reversing a direction vector of each triangular mesh, and the sizes of the replicated spheres are gradually increased in the process of generating the plurality of spheres in a distributed manner so that representative spheres are evenly arranged on individual teeth.

According to the performance of the process, spheres corresponding to 14 teeth of each of a lower jaw and an upper jaw are generated, and thereafter, when the coordinates represented by the spheres are derived, representative coordinates of each tooth are derived, and a contact point of the teeth is derived through the contact point of the spheres, so that the coordinates of the teeth and feature data for the contact point may be extracted.

In addition, according to the present invention, information about the position, rotation, and size of the teeth may be extracted based on the coordinates of the representative sphere, and axis data for each tooth may be generated so as to be parallel to a root direction of the teeth, thereby securing individual feature data of each tooth and completing the object classification between the tooth part (30T) and the gum part (30G) of the subject.

Meanwhile, according to the present invention, it is possible to derive an image of each individual tooth through a mesh separation program based on a SplitDisjointMesh function in a program such as an Rhino 3D work tool, and it is possible to generate data including each tooth data, feature data of teeth, and an axis of teeth by processing a tooth image by deriving the axis of teeth based on mesh information for each tooth for the tooth image, and it is possible to clearly identify an object corresponding to the tooth part (30T) by automatically deriving a volume center point and an area center point from the mesh information and setting the axis of the teeth.

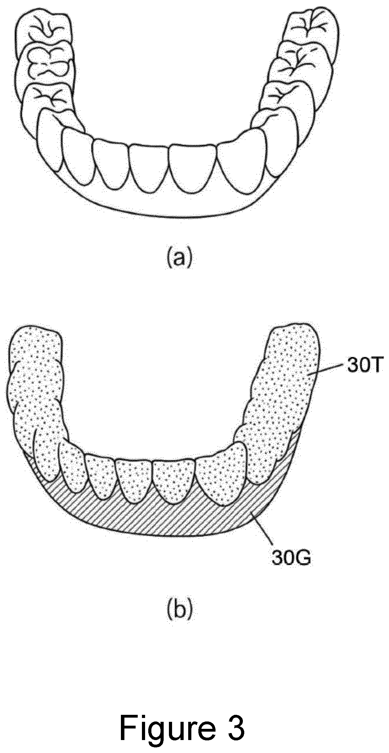

Meanwhile, FIG. 3(a) shows an example of the oral scan data (1) about the lower jaw among the oral scan data (1)of the subject, and FIG. 3(b) shows an example of the tooth part (30T) and the gum part (30G) for the lower jaw of the subject, which are separated and identified using the object extraction algorithm.

Obviously, according to another embodiment of the present invention, various known technologies such as a technology of dividing a boundary between the tooth part (30T) and the gum part (30G) using a machine learning-based 3D object extraction algorithm (CNN, PointNetemd) and an edge detection algorithm may be applied, but the present invention is not limited thereto.

As described in a temporary model generation step (S10), according to the present invention, when the identification of the teeth and the gums of the subject from the oral scan data (1) of the subject is completed by the object extraction algorithm, the basic shape of the mouth shield (30) is designed using a design program based on data for the identified teeth and gums.

As one embodiment, the above-described design program may utilize CAD/CAM software, and non-uniform rational B-splines (NURBS)-based surface modeling may be performed to generate a temporary model of the mouth shield (30)that is customized by reflecting an oral structure of the subject.

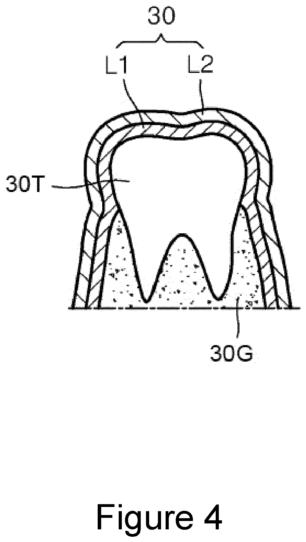

In addition, as a preferred embodiment, when the above-described temporary model is generated, as a design material of the temporary model, a urethane-based material is used in a form of multi-layers. Specifically, as shown in FIG. 4, the design material includes a first layer (L1) that directly surrounds an outer surface of the tooth part (30T) of the subject, and a second layer (L2) that surrounds an outer surface of the first layer (L1).

In this case, the above-described first layer (L1) is formed of a urethane-based material having elastic modulus and hardness that are relatively lower than those of the second layer (L2),and the second layer (L2)is formed of a urethane-based material having elastic modulus and hardness that are relatively higher than those of the first layer (L1).

That is, according to the present invention, the mouth shield(30)having urethane-based materials with mutually different physical properties is designed using the fact that even the same urethane may be designed to have different physical properties according to a curing method and a molecular structure (crosslinking density).

As one embodiment, according to the present invention, the first layer (L1)may be formed of urethane having a hardness of about 70 to 90 based on a Shore A hardness, and the second layer (L2)may be formed of urethane having a hardness of about 30 to 80 based on a Shore D hardness.

That is, according to the present invention, it may be understood that the mouth shield (30)is designed such that the first layer (L1), which is a portion directly in contact with the tooth part (30T), is formed of relatively soft urethane, and the second layer (L2) constituting the outer surface of the mouth shield (30) is formed of relatively hard urethane, thereby ensuring durability while improving wearing sensation when the subject wears the mouth shield (30).

Meanwhile, the first layer (L1) and the second layer (L2) having different physical properties may be implemented in a form of multi-layers by applying an SLA-type biocompatible resin printing to output the first layer (L1), and continuously stacking the second layer (L2), and an interlayer bonding strength may be increased through a UV post-curing process as a post-treatment process.

Obviously, as another embodiment of stacking the multi-layers, according to the present invention, the first layer (L1) and the second layer (L2) may be separately manufactured and chemically bonded to each other by a chemical bonding method such as plasma treatment, so that the first layer (L1) and the second layer (L3) are assembled into one mouth shield (30), but the present invention is not limited thereto.

Meanwhile, after the temporary model generation step (S10) of FIG. 1, a definitive model generation step (S20)of generating a definitive model by performing correction on the temporary model generated in temporary model generation step (S10), in which the definitive model is generated by forming continuous concave groove parts (H)in an inner region including at least one of a lingual side and a labial side of the temporary model along an arrangement direction of teeth, is performed.

Specifically, in definitive model generation step (S20), in forming the concave groove parts (H) in the temporary model, the concave groove parts (H) are formed based on a gingival margin line corresponding to a boundary site between the tooth part (30T) and the gum part (30G) in the temporary model. Meanwhile, the concave groove part (H) may be understood as performing a structural role for stably injecting a medicament (e.g., antibiotics, anti-inflammatory agents, etc.) for enhancing the health of the oral cavity.

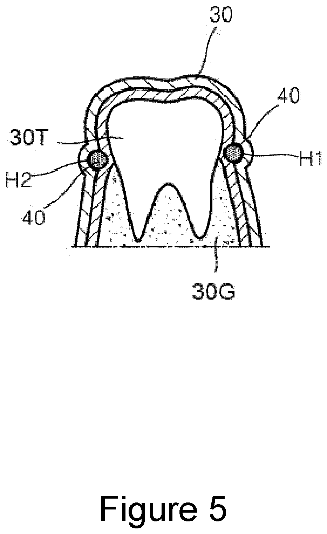

As a first embodiment of generating the concave groove parts (H) in the temporary model, continuing the description with reference to FIG. 5, in definitive model generation step (S20), the definitive model may be generated by designing a structure in which, when the concave groove parts (H) are formed in the temporary model, a concave groove part (H1) having a predetermined depth is formed in a lingual side or palatal side (i.e., that inner surface of the teeth of the upper and lower jaws) based on the gingival margin line, and a concave groove part (H2) having a predetermined depth is formed in a labial side or buccal side (i.e., the outer surface of the teeth).

That is, it may be understood that the first embodiment is an embodiment in which the concave groove part (H) is formed in a region corresponding to inner and outer surfaces of the teeth in a boundary surface of the mouth shield (30) in contact with the tooth part (30T), respectively, so that the medicament supply unit is installed in a region adjacent to a gingival margin part, thereby allowing the medicament supply unit installed in the concave groove part (H) to effectively inject the medicament along the gingival margin line of the subject.

In addition, as a second embodiment of generating the concave groove parts (H) in the temporary model, according to the present invention, the definitive model may be generated by designing the same such that the concave groove parts (H) are formed along the gingival margin line, in which a concave groove part (H) having a predetermined depth is formed in the lingual side or palate side, and a concave groove part (H) has a shape that does not have a separate concave groove part (H) in the labial side or buccal side.

That is, according to the second embodiment described above, the installation region of the medicament supply unit is limited to the inner surface of the tooth part (30T), and even if the subject performs external activities while wearing the mouth shield (30), the process of supplying the medicament to the gingival margin part in the concave groove parts (H) formed in the mouth shield (30) and the mouth shield (30) is not noticeable, thereby promoting excellent aesthetics and oral health enhancement due to the medicament supply.

In addition, in definitive model generation step (S20), when a medicament to be prescribed to the subject is determined during the formation of the concave groove parts (H), the required amount of the medicament may be calculated in consideration of factors including at least one of age, body weight, gum condition, and severity of a disease of the subject, and a concave groove part H having a size corresponding to the calculated required amount of the medicament may be designed.

In this case, it may be understood that the reason for considering the age of the subject described above is that the tissue condition of the gums and the periodontal varies depending on children, adolescents, adults, and the elderly, and it may be understood that the reason for considering the body weight is that the absorption and metabolism rate of the medicament may vary depending on the body weight.

In addition, considering the gum condition may be understood as being because a medicament of a specific component is required when the subject has gum diseases such as gingivitis and periodontitis, and it may be understood as a configuration for providing a personalized medicament supply unit to the subject by considering that the concentration of the medicament may vary depending on the initial, intermediate, and terminal stages.

As a more specific embodiment to the description, according to the present invention, oral examination data for the subject may be additionally utilized, and an appropriate amount of the medicament may be calculated based on the oral examination data of a patient.

For example, in the case of a subject who has a gum disease and has a relatively severe severity of the gum disease, the size of the concave groove part (H) may be adjusted to be relatively larger than the predetermined size, and in the case of a subject who is of a low age, the size of the concave groove part (H) may be made smaller than the predetermined size to reduce the amount of the medicament because the absorption amount of the medicament may be high.

Meanwhile, the size of the concave groove part (H) may be adjusted such that the depth and size of the concave groove part (H) are set to be deeper and wider than the set value as the required amount of the medicament is larger, and the depth and size of the concave groove part (H) are set to be shallower and narrower than the set value as the required amount of the medicament is smaller.

That is, according to the present invention, since it is possible to generate a definitive model of the mouth shield (30) capable of precisely adjusting the amount of the medicament according to the oral condition of the subject, it is possible to prevent excessive use of the medicament and minimize side effects, and the concave groove part (H) is designed to be personalized, thereby providing the mouth shield (30) having high utilization as an auxiliary device for orthodontic treatment or periodontal disease treatment.

In addition, as another embodiment of definitive model generation step (S20), according to the present invention, when the concave groove parts (H) are formed, mutually different design factors may be reflected for each region of the inner surface of the concave groove part (H).

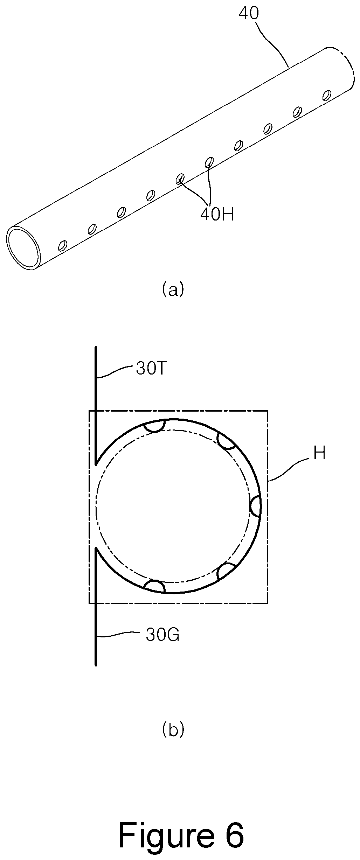

As an example, referring to FIG. 6, according to the present invention, a low friction coating is applied to an inlet region of the concave groove part (H) and a high friction protrusion pattern is applied to an inner region of the concave groove (H), so that when a dedicated tube (40) is installed as the medicament supply unit installed in the concave groove part (H), an outer skin of the dedicated tube (40) is designed to have a constant frictional force with an inside of the concave groove part (H), thereby stably fixing a gel tube when the medicament is supplied using the dedicated tube (40), but easily separating the gel tube from the concave groove part (H) when the dedicated tube (40) is replaced.

To this end, according to the present invention, a fluorine-based coating is applied to the concave groove part (H) by a spray method in order to induce smooth insertion of the dedicated tube (40), which is the medicament supply unit, into the inlet region of the concave groove part (H), that is, a region adjacent to the open surface, and a friction force is minimized by maintaining a surface roughness at a level of 0.1 to 0.3 µm, thereby improving convenience of separation for insertion installation or replacement of the dedicated tube (40).

In addition, as schematically shown in the sectional view of FIG. 6(b) (in this case, 30T refers to a surface on which the mouth shield is in contact with the tooth part (30T)and 30G refers to a surface on which the mouth shield is in contact with the gum part (30G)), a plurality of fine protrusion patterns may be formed in the inner region except for the inlet region of the concave groove part (H) to increase a contact area between end portions of the fine protrusions and the outer skin of the dedicated tube (40), thereby designing a structure that may prevent the dedicated tube (40) coupled to the concave groove part (H) from being easily separated and enables replacement without unnecessarily applying a large force to the replacement after removing the dedicated tube (40) from the concave groove part (H) when replacement of the dedicated tube (40) is required due to exhaustion of the medicament.

For example, although the density of the protrusion pattern may be adjustable according to the material and surface characteristics of the tube, the SLA-based 3D printing may be used to uniformly arrange protrusions having a size of 50 to 100 µm to increase the frictional force with the outer surface of the dedicated tube (40), thereby implementing the shape of the mouth shield (30) having a structure in which the dedicated tube (40) may be easily separated when necessary without shaking upon installation of the dedicated tube (40).

That is, in definitive model generation step (S20) described above, it will be understood that a process of deriving a definitive model provided with the medicament supply unit by modifying the internal shape of the mouth shield (30), which is generated as the temporary model, into an optimal shape in which the medicament supply unit may be installed is performed.

Meanwhile, design data for the definitive model generated in definitive model generation step (S20) is preferably provided to a 3D printing laboratory.

In this case, as described above, a 3D printing laboratory (20) may be understood as a concept of a dedicated production facility for manufacturing a customized medical device using a 3D printer (21). However, the 3D printing laboratory (20) needs to be understood as a facility for manufacturing a medical device with high precision using a medical material, rather than simply manufacturing a real device using the 3D printer (21).

More specifically, it is preferable that the design data for the definitive model designed in definitive model generation step (S20) is converted into a format optimized for 3D printing and transmitted to the 3D printing laboratory (20), and the format optimized for 3D printing described above may be understood as a concept of a format including at least one of STL, OBJ, and AMF.

That is, based on the design data for the definitive model derived by the performance of definitive model generation step (S20), the real mouth shield (30) provided with the concave groove part (H), which is the medicament supply unit, is manufactured as the mouth shield (30) personalized to the patient in the 3D printing laboratory, and the real mouth shield (30) is provided to the patient, so that it is possible to provide a mouth shield product optimized for the individual state of the subject.

Meanwhile, as described above, the mouth shield (30) in which the concave groove part (H) is formed may apply or insert the medicament into the concave groove part (H) described above to deliver the medicament to a target site (i.e., the gingival margin part) of the subject only by the subject who wears the mouth shield in the oral cavity.

As one embodiment, according to the present invention, in order to supply the medicament through the concave groove part, the solidified medicament may be inserted into the concave groove part, and when the subject wears the real mouth shield in the oral cavity, the solidified medicament may be gradually dissolved by an oral environment (intra-oral temperature, saliva, etc.) so that the medicament may be supplied to the subject.

In this case, a gel-type medicament may be simply applied to the concave groove part (H), but preferably, according to the present invention, a medicament including fluorine for preventing tooth decay, and a medicament including a component for preventing or improving periodontitis and gingivitis may be solidified using a polymer matrix (a method of mixing a biocompatible polymer with PEG and PLGA to convert the same into a solid formulation) or the like, and then the medicament may be slowly discharged after being inserted into the concave groove part, or a sustained-release tablet may be compression-molded (a method of mixing the gel-type medicament with an absorbent and granulating the same to refine or encapsulate the same) according to the shape of the concave groove part (H) of the mouth shield (30), and then inserted into the concave groove part, so that when the mouth shield (30) is inserted into the oral cavity of the subject, the compressed and molded medicament may be gradually dissolved by saliva of the subject and the medicament may be delivered to the target site of the subject for a predetermined time.

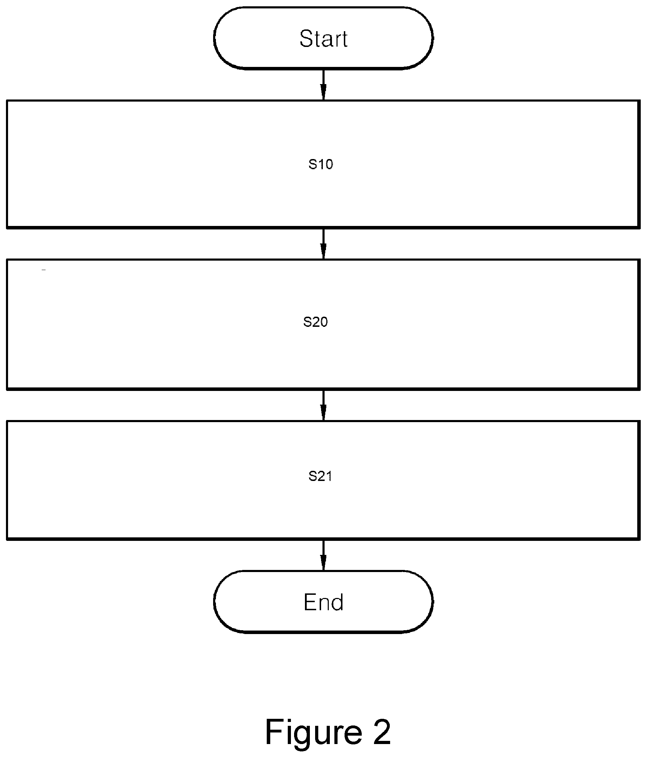

In this case, as another embodiment of the preset invention, simultaneously referring to FIG. 2, the present invention may further include a dedicated tube designing step (S21) of installing a dedicated tube that is designed in a shape corresponding to the concave groove part as the medicament supply unit and has a discharge structure for discharging the gel-type medicament that is filled in the dedicated tube, after definitive model generation step (S20) described above.

Specifically, in dedicated tube designing step (S21) described above, after the dedicated tube (40) is filled with the gel-type medicament, the dedicated tube (40) filled with the medicament may be detachably installed on the concave groove part (H), and as shown in FIG. 6(a), a first dedicated tube may be designed to have a plurality of perforated holes (40H) formed in the outer surface of the dedicated tube (40) as a discharge path through which the medicament filled in the dedicated tube (40) is discharged.

As one embodiment, a material of the dedicated tube (40) may be a silicone-based material, and an outer shape of the dedicated tube (40) is automatically designed to match the shape of the concave groove part (H).

In addition, the dedicated tube (400) is provided therein with a filling space capable of storing a medical gel-type medicament, and the medicament to be filled may be components including antibiotics, anti-inflammatory agents, fluorogels, and analgesics.

In addition, the plurality of perforated holes (40H) formed in the dedicated tube (40) may be understood as a unit for discharging the medicament filled therein and a configuration for adjusting the discharge rate of the medicament, and preferably, the gel-type medicament is slowly discharged by setting the size of the perforated hole in a range of 50 to 300 µm (micrometer).

The perforated hole (40H) may be formed in the entire outer surface of the dedicated tube, or a formation position of the perforated hole (40H) may be limited to a region limited to a local site corresponding to the gingival margin line as shown in FIG. 5(a), but the present invention is not limited thereto.

Meanwhile, when the medicament filled in the dedicated tube (40) is simply filled in the dedicated tube (40) in which the plurality of perforated holes (40H) are formed, there is a concern that the medicament may be filled and discharged through the perforated holes (40H) at the same time. Accordingly, according to the present invention, in dedicated tube designing step (S21), a second dedicated tube may be designed based on the previously designed first dedicated tube, in which a thin film-type elastic film including at least one of silicone, polyurethane, and rubber is provided inside the perforated holes (40H) to block the medicament from being discharged initially, and after the dedicated tube (40) is installed in the concave groove part (H), when the subject wears the mouth shield (30), which is provided with the dedicated tube (40) as the medicament supply unit, in the oral cavity, the elastic film is deformed as a pressure is applied into the oral cavity to allow the medicament to be gradually discharged through the perforated holes (40H).

Obviously, in another embodiment, according to the present invention, in dedicated tube designing step (S21), a third dedicated tube may be designed based on the previously designed first dedicated tube, in which the third dedicated tube has a structure in which, when the dedicated tube (40) is mounted in the concave groove part (H) of the mouth shield (30) and worn in the oral cavity of the subject, the outer surface of the dedicated tube (40) is provided in a sealed state by the dissolving film, and the dissolving film is gradually dissolved and disappears due to contact with humidity and saliva in the oral cavity, thereby naturally discharging the medicament through the perforated hole (40H).

In this case, as the dissolving film usable in the dedicated tube (40), a dissolving film including at least one of colorless and odorless hydroxypropyl methylcellulose having high biocompatibility and high biocompatibility, polyvinyl alcohol having high biocompatibility and well-dissolved in water, gelatin having an excellent binding force with the medicament and excellent biocompatibility, carboxymethylcellulose commonly used as a dental and medical coating agent, and non-toxic and naturally decomposable pullulan, which is eco-friendly, may be suitably used.

That is, according to the present invention, the dedicated tube (40) is designed to include at least one of the first dedicated tube, the second dedicated tube, and the third dedicated tube described above is designed in dedicated tube designing step (S21), so that the problem of medicament leakage at an undesired time point is fundamentally blocked before the dedicated tube (40) is mounted on the mouth shield (30), and only when the mouth shield (30) is worn in the oral cavity of the subject, the perforated holes (40H) are opened by the pressure in the oral cavity or the environmental characteristics of the oral cavity to gradually and continuously supply the medicament to the gingival margin part of the subject, thereby achieving the purpose of enhancing the oral health of the subject, but the present invention is not limited thereto.

Meanwhile, according to the present invention, when dedicated tube designing step (S21) is performed after definitive model generation step (S20), the design data for the definitive model and design data for the dedicated tube (40) may be provided to the 3D printing laboratory (20) to manufacture the real mouth shield (30) and the dedicated tube (40) capable of filling with the gel-type medicament, and the mouth shield (30) provided with the dedicated tube (40) filled with the medicament may be provided to the subject, and due to steps S10 to S31, the present invention may provide a mouth shield with improved wearing sensation in the oral cavity and durability while achieving the purpose of enhancing health by the medicament by continuously supplying the medicament, which is prepared to enhance the oral health, to the specific site.

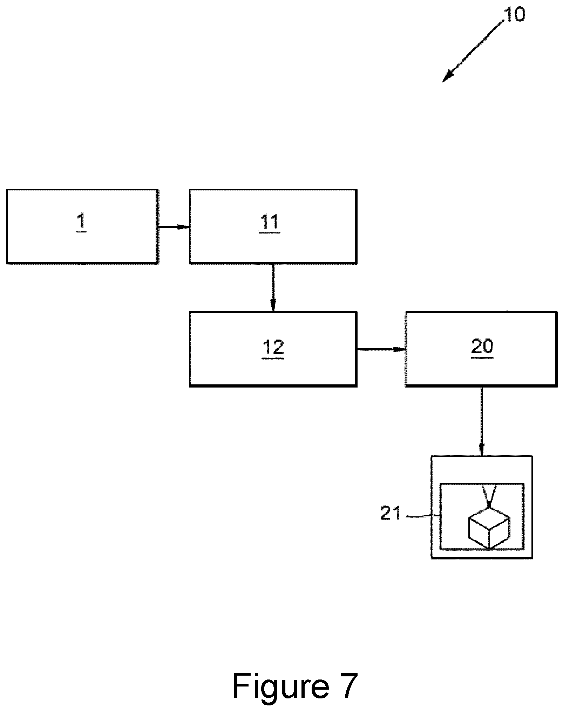

On the other hand, referring to 10 of FIG. 7, it can be seen that FIG. 7 shows a configuration view of an apparatus (10) for designing the mouth shield (30) having the medicament supply unit according to one embodiment of the present invention.

Specifically, the apparatus (10) described in the present invention includes a temporary model generation unit (11) and a definitive model generation unit (12) as main components.

In this case, the temporary model generation unit (11) serves to identify a tooth part (30T) and a gum part (30G) using a preset object extraction algorithm from oral scan data (1), which is obtained by three-dimensionally scanning an oral condition of a subject, and to generate a temporary model by performing modeling on a mouth shield (30) having a shape corresponding to the identified tooth part (30T) and gum part (30G) for the subject using a design program, and as a result, it may be understood that the temporary model generation unit 11 may perform all functions performed in step S10 of FIG. 1.

In addition, the definitive model generation unit (12) serves to generate the definitive model by performing correction on the temporary model generated by the temporary model generation unit (11), and to generate the definitive model by forming continuous concave groove part H in an inner region of the oral cavity of the temporary model along an arrangement direction of the teeth.

That is, it may be understood that the definitive model generation unit (12) may perform all the functions performed in definitive model generation step (S20) of FIG. 1 described above, and the description of the overlapping embodiments described above will be omitted.

Meanwhile, although not explicitly shown in FIG. 7, the apparatus (10) configuration of the present invention may further include a dedicated tube design unit. In this case, the dedicated tube design unit is designed in a shape corresponding to the concave groove part H as the medicament supply unit, and serves to design the dedicated tube (40) having a discharge structure for discharging a gel-type medicament filled in the dedicated tube (40), and as a result, it may be understood that all functions performed by dedicated tube designing step (S21) of FIG. 2 may be performed.

According to one embodiment of the present invention described comprehensively above, a medicament that is prepared to enhance oral health in the oral cavity is continuously supplied to a specific site, so that a treatment effect may be maintained for a long time compared to a simple gargle or ointment application method, and accordingly, the purpose of enhancing oral health may be achieved rapidly.

In addition, according to one embodiment of the present invention, an efficient local treatment effect may be expected by forming the concave groove part formed in the mouth shield along the gingival margin line, and an excessive supply of a medicament and an appropriate treatment effect may be expected by adjusting the amount and discharge rate of the medicament.

In addition, according to one embodiment of the present invention, a material of the mouth shield is implemented in a form of multi-layers formed of soft urethane and hard urethane, it is possible to expect improvement in wearing sensation and durability of the mouth shield.

In addition, according to one embodiment of the present invention, it is possible to provide an optimal treatment solution by enabling customized manufacture of a mouth shield reflecting an oral condition and an oral structure of a subject, and to provide a mouth shield having various applicability, from a preventive function of tooth decay prevention, treatment of periodontal disease, pain relief, and whitening treatment, to a cosmetic purpose, treatment of a disease, and the like, according to the characteristics of the medicament.

While the embodiments have been described with reference to limited examples and drawings as described above, it will be apparent to one of ordinary skill in the art that various changes and modifications may be made from the above description.

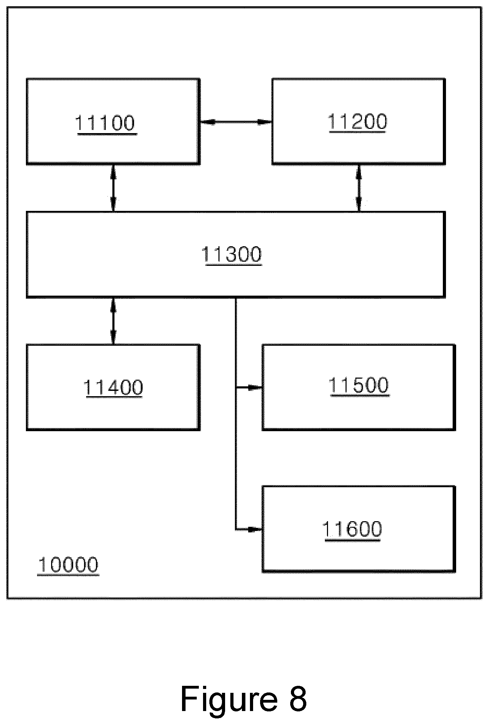

Meanwhile, referring to FIG. 8, FIG. 8 is a view showing an example of an internal configuration of the computing device according to one embodiment of the present invention. In the following description, redundant descriptions of the embodiment corresponding to the above descriptions for FIGS. 1 to 7 will be omitted.

As shown in FIG. 8, a computing device (10000) may at least include at least one processor (11100), a memory (11200), a peripheral interface (11300), an input/output (I/O) subsystem (11400), a power circuit (11500), and a communication circuit (11600). In this case, the computing device (10000) may correspond to a user terminal A connected to a tactile interface device or correspond to the above-described computing device B.

The memory (11200) may include, for example, a high-speed random access memory, a magnetic disk, an SRAM, a DRAM, a ROM, a flash memory, or a non-volatile memory. The memory (11200) may include a software module, an instruction set, or other various data necessary for the operation of the computing device (10000).

In this case, access to the memory (11200) from other components of the processor (11100) or the peripheral interface (11300), may be controlled by the processor (11100).

The peripheral interface (11300) may combine an input and/or output peripheral device of the computing device (10000) to the processor (11100) and the memory (11200). The processor (11100) may execute the software module or the instruction set stored in the memory (11200), thereby performing various functions for the computing device (10000) and processing data.

The input/output subsystem (11400) may combine various input/output peripheral devices to the peripheral interface (11300). For example, the input/output subsystem (11400) may include a controller for combining the peripheral device such as monitor, keyboard, mouse, printer, or a touch screen or sensor, if needed, to the peripheral interface (11300). According to another aspect, the input/output peripheral devices may be combined to the peripheral interface (11300) without passing through the input/output subsystem (11400).

The power circuit (11500) may provide power to all or a portion of the components of the terminal. For example, the power circuit (11500) may include a power failure detection circuit, a power converter or inverter, a power status indicator, a power failure detection circuit, a power converter or inverter, a power status indicator, or arbitrary other components for generating, managing, or distributing power.

The communication circuit (11600) may use at least one external port to enable communication with other computing devices.

Alternatively, as described above, the communication circuit (11600) may include an RF circuit, if needed, to transmit and receive an RF signal, also known as an electromagnetic signal, thereby enabling communication with other computing devices.

The above embodiment of FIG. 8 is merely an example of the computing device (10000), and the computing device (11000) may have a configuration or arrangement in which some components shown in FIG. 8 are omitted, additional components not shown in FIG. 8 are further provided, or at least two components are combined. For example, a computing device for a communication terminal in a mobile environment may further include a touch screen, a sensor, or the like, in addition to the components shown in FIG. 8. The communication circuit (11600) may include a circuit for RF communication of various communication schemes (such as WiFi, 3G, LTE, Bluetooth, NFC, and Zigbee). The components that may be included in the computing device (10000) may be implemented by hardware, software, or a combination of both hardware and software which include at least one integrated circuit specialized in a signal processing or an application.

The methods according to the embodiments of the present invention may be implemented in the form of program instructions to be executed through various computing devices so as to be recorded in a computer-readable medium. In particular, a program according to the embodiment of the present invention may be configured as a PC-based program or an application dedicated to a mobile terminal. The application to which the present invention is applied may be installed in a user terminal through a file provided by a file distribution system. For example, a file distribution system may include a file transmission unit (not shown) that transmits the file according to the request of the user terminal.

The above-described device may be implemented by hardware components, software components, and/or a combination of hardware components and software components. For example, the devices and components described in the embodiments may be implemented by using at least one general purpose computer or special purpose computer such as a processor, a controller, an arithmetic logic unit (ALU), a digital signal processor, a microcomputer, a field programmable gate array (FPGA), a programmable logic unit (PLU), a microprocessor, or any other device capable of executing and responding to instructions. The processing device may execute an operating system (OS) and one or more software applications executed on the operating system.

In addition, the processing device may access, store, manipulate, process, and create data in response to the execution of the software. For the further understanding, in some cases, one processing device may be used, however, those skilled in the art will be appreciated that the processing device may include a plurality of processing elements and/or a plurality of types of processing elements. For example, the processing device may include a plurality of processors or one processor and one controller. In addition, other processing configurations, such as a parallel processor, are also possible.

The software may include a computer program, a code, an instruction, or a combination of at least one thereof, may configure the processing device to operate as desired, or may instruct the processing device independently or collectively. In order to be interpreted by the processor or to provide instructions or data to the processor, the software and/or data may be permanently or temporarily embodied in any type of machine, component, physical device, virtual equipment, and computer storage medium or device. The software may be distributed over computing devices connected to networks, so as to be stored or executed in a distributed manner. The software and data may be stored in at least one computer-readable recording medium.

The above-described embodiments of the present invention may be recorded in computer-readable media including program instructions to implement various operations embodied by a computer. The computer-readable medium may also include, alone or in combination with the program instructions, data files, data structures, and the like. The program instructions recorded on the media may be those specially designed and constructed for the purposes of the embodiments, or they may be of the kind well-known and available to those having skill in the computer software arts. Examples of computer-readable recording media include magnetic media such as hard disks, floppy disks, and magnetic tape; optical media such as CD ROM disks and DVDs; magneto-optical media such as optical discs; and hardware devices that are specially configured to store and perform program instructions, such as read-only memory (ROM), random access memory (RAM), flash memory, and the like.

Examples of program instructions include both machine code, such as produced by a compiler, and files containing higher level code that may be executed by the computer using an interpreter. The described hardware devices may be configured to act as one or more software modules in order to perform the operations of the above-described embodiments of the present invention, or vice versa.

While the embodiments have been described with reference to limited examples and drawings as described above, it will be apparent to one of ordinary skill in the art that various changes and modifications may be made from the above description. Suitable results may be achieved if the described techniques are performed in a different order, and/or if components in a described system, architecture, device, or circuit are combined in a different manner, and/or replaced or supplemented by other components or their equivalents. Therefore, other implementations, other embodiments, and equivalents of the claims are within the scope of the following claims.

### REFERENCE NUMBERS

1 Oral scan data
10 Apparatus
11 Temporary model generation unit
12 Definitive model generation unit
20 3D printing laboratory
21 3D printer
30 Mouth shield
30T Tooth part
30G Gum part
40 Dedicated tube
40H Perforated hole
10000 Computing device
11100 Processor
11200 Memory
11300 Peripheral interface
11400 Input/output (I/O) subsystem
11500 Power circuit
11600 Communication circuit
S10 Temporary model generation step
S20 Definitive model generation step
S21 Dedicated tube designing step
H Concave groove parts
H1 Concave groove part (in a lingual side or palatal side)
H2 Concave groove part (in a labial side or buccal side)
L1 First layer
L2 Second layer

## Claims

1. A method for designing a mouth shield having a medicament supply unit, which is implemented by a computing device (10000) including one or more processors (11100) and one or more memories (11200) for storing instructions executable in the (11100) processors, the method comprising:
a temporary model generation step (S10) of identifying a tooth part (30T) and a gum part (30G) using a preset object extraction algorithm from oral scan data (1), which is obtained by three-dimensionally scanning an oral condition of a subject, and generating a temporary model by performing modeling on the mouth shield (30) having a shape corresponding to the identified tooth part (30T) and gum part (30G) for the subject using a design program; and
a definitive model generation step (S20) of generating a definitive model by performing correction on the temporary model generated in the temporary model generation step (S10), in which the definitive model is generated by forming continuous concave groove parts in an inner region including at least one of a lingual side and a labial side of the temporary model along an arrangement direction of teeth,
wherein design data for the definitive model generated in the definitive model generation step (S20) is provided to a 3D printing laboratory (20),
wherein, in the definitive model generation step (S20), when the concave groove parts are formed, the concave groove parts (H) are formed based on a gingival margin line corresponding to a boundary site between the tooth part (30T) and the gum part (30G) in the temporary model, and
wherein, when the concave groove parts (H) are formed, a required amount of a medicament is calculated in consideration of factors including at least one of age, body weight, gum condition, and severity of a disease of the subject, and a concave groove part (H) having a size corresponding to the calculated required amount of the medicament is designed.

2. The method of claim 1, further comprising a dedicated tube designing step (S21) of designing a dedicated tube (40) that is designed in a shape corresponding to the concave groove part (H) as the medicament supply unit and has a discharge structure for discharging a gel-type medicament that is filled in the dedicated tube (40), after the definitive model generation step (S20),
so that the design data for the definitive model and design data for the dedicated tube (40) are provided to the 3D printing laboratory (20) to manufacture a real mouth shield and a dedicated tube (40) that enables filling of the gel-type medicament.

3. The method of claim 2, wherein in the dedicated tube (40) designing step,
the medicament is automatically designed such that, after the dedicated tube (40) is filled with the gel-type medicament, the dedicated tube (40) filled with the medicament matches a shape of the concave groove part (H) to allow the dedicated tube (40) to be detachably installed on the concave groove part (H), and
the dedicated tube (40) is designed to include at least one of a first dedicated tube formed on an outer surface thereof with a plurality of perforated holes serving as a discharge path for discharging a medicament filled in the dedicated tube (40) such that the filled medicament is discharged, a second dedicated tube designed based on the first dedicated tube, in which the perforated holes (40H) are additionally provided with a thin film-type elastic membrane so that when the subject wears the mouth shield provided with the dedicated tube (40) in the oral cavity, the thin film-type elastic membrane is deformed due to pressure inside the oral cavity so that the filled medicament is discharged, and a third dedicated tube designed based on the first dedicated tube, in which the outer surface of the dedicated tube (40) is sealed with a dissolving film so that when the subject wears the mouth shield (30) provided with the dedicated tube (40) in the oral cavity, the dissolving film is dissolved so that the filled medicament is discharged through the perforated holes (40H).

4. The method of claim 1, wherein in the temporary model generation step (S10),
a urethane-based material is used in a form of multi-layers as a material of designing the temporary model,
in which the multi-layers include a first layer configured to directly surround an outer surface of the tooth part (30T) of the subject, and a second layer configured to surround an outer surface of the first layer,
the first layer is formed of a urethane-based material having elastic modulus and hardness that are relatively lower than elastic modulus and hardness of the second layer, and
the second layer is formed of a urethane-based material having elastic modulus and hardness that are relatively higher than the elastic modulus and hardness of the first layer.

5. The method of claim 1, wherein in the definitive model generation step (S20),
mutually different design factors are applied for each region of an inner surface of the concave groove part (H),
a low friction coating is applied to an inlet region of the concave groove part (H), and
a high friction protrusion pattern is applied to an inner region of the concave groove part (H).

6. An apparatus (10) for designing a mouth shield (30) having a medicament supply unit, which is implemented by a computing device (10000) including one or more processors (11100) and one or more memories (11200) for storing instructions executable in the processors (11100), the apparatus (10) comprising:
a temporary model generation unit (11) configured to identify a tooth part (30T) and a gum part (30G) using a preset object extraction algorithm from oral scan data (1), which is obtained by three-dimensionally scanning an oral condition of a subject, and generate a temporary model by performing modeling on the mouth shield having a shape corresponding to the identified tooth part (30T) and gum part (30G) for the subject using a design program; and
a definitive model generation unit (12)configured to generate a definitive model by performing correction on the temporary model generated by the temporary model generation unit (11), in which the definitive model is generated by forming continuous concave groove parts (H) in an inner region of the temporary model along an arrangement direction of teeth,
wherein design data for the definitive model generated by the definitive model generation unit (12) is provided to a 3D printing laboratory (20),
wherein the definitive model generation unit (12) forms the concave groove parts (H) are formed based on a gingival margin line corresponding to a boundary site between the tooth part (30T) and the gum part (30G) in the temporary model, when the concave groove parts (H) are formed, and
wherein, when the concave groove parts (H) are formed, a required amount of a medicament is calculated in consideration of factors including at least one of age, body weight, gum condition, and severity of a disease of the subject, and a concave groove having a size corresponding to the calculated required amount of the medicament is designed.

7. A computer-readable recording medium that stores instructions for allowing a computing device (10000) to perform the following steps, wherein the steps comprise:
a temporary model generation step (S10) of identifying a tooth part (30T) and a gum part (30G) using a preset object extraction algorithm from oral scan data (1), which is obtained by three-dimensionally scanning an oral condition of a subject, and generating a temporary model by performing modeling on the mouth shield having a shape corresponding to the identified tooth part (30T) and gum part (30G) for the subject using a design program; and
a definitive model generation step of generating a definitive model by performing correction on the temporary model generated in the temporary model generation step (S10), in which the definitive model is generated by forming continuous concave groove parts (H) in an inner region including at least one of a lingual side and a labial side of the temporary model along an arrangement direction of teeth,
wherein design data for the definitive model generated in the definitive model generation step (S20) is provided to a 3D printing laboratory (20),
wherein, in the definitive model generation step (S20), when the concave groove parts (H) are formed, the concave groove parts are formed based on a gingival margin line corresponding to a boundary site between the tooth part (30T) and the gum part (30G) in the temporary model, and
wherein, when the concave groove parts (H) are formed, a required amount of a medicament is calculated in consideration of factors including at least one of age, body weight, gum condition, and severity of a disease of the subject, and a concave groove having a size corresponding to the calculated required amount of the medicament is designed.
